# EUROPEAN PATENT APPLICATION

(11) **EP 0 567 738 A2**
(43) Date of publication of application: **03.11.1993**
(21) Application number: 93103107.4
(22) Date of filing: 26.02.1993
(51) Int. Cl.: C12M 1/34, C12N 5/00

(54) **Controlling perfusion rates in continuous bioreactor culture of animal cells**

(30) Priority: 01.05.1992 US 877900
(71) Applicant: AMERICAN CYANAMID COMPANY, Wayne, NJ 07470-8426 (US)
(72) Inventor: Magargal II, Wells Wrisley, Nyack, New York 10960 (US)
(74) Representative: Wächtershäuser, Günter, Prof. Dr.

(57) **Abstract**

The invention is a method for controlling the perfusion rate of bioreactor cultures of animal cells, wherein the level of metabolic activity of the cells, as reflected by a drop in pH and as measured by a pH probe, is used to activate 1) the perfusion of fresh medium containing standard cell nutrients and experimentally determined concentrations of energy source(s) and buffer, into the bioreactor and 2) the removal of culture fluid, which is depleted of nutrients and contains waste products, from the bioreactor.

## Description

### SUMMARY OF THE INVENTION

The invention is a method for controlling perfusion rates in a continuous bioreactor culture of animal cells requiring pH maintenance which comprises:
a) adding fresh medium enriched with glucose, glutamine and bicarbonate to the culture medium in the bioreactor when the culture medium pH drops below setpoint;
b) discontinuing the addition of the enriched medium when the culture medium pH reaches setpoint; and
c) repeating steps (a) and (b) on a continuous basis to maintain the desired setpoint pH.

For example, a culture of Vero cells grown on microcarriers, which use glucose and glutamine as their major energy sources, can be perfused with standard cell culture medium containing concentrations of glutamine, glucose and bicarbonate which are experimentally determined, such that when perfusion is activated by a signal from a pH probe as pH drops below a setpoint, the levels of nutrients and waste products are maintained at concentrations which promote the growth and viability of the cells.

### BACKGROUND OR FIELD OF THE INVENTION

The invention described herein relates to the in vitro cultivation of animal cells in perfusion bioreactors and more particularly to the control of perfusion rates in bioreactor cultures of anchorage dependent or suspension animal cells.

The need for economical and consistent cultures of animal cells for the production of vaccines, monoclonal antibodies and recombinant proteins is increasing. The recognition that animal cells offer the best environment for the production of biological products which are identical or most similar to those actually employed by animals, or which, in the case of vaccines, give the broadest and strongest immune response, has fueled the need for systems which can grow animal cells and produce their products consistently and economically.

To maintain appropriate metabolite concentrations in bioreactors, perfusion rates of fresh medium into the bioreactor are typically determined by off line measurement of nutrients and waste products. Manual adjustment of the perfusion rate is made based on these measurements. The pH of the culture is maintained by the separate addition of a concentrated solution of buffer either manually or automatically in response to a signal from a pH probe in the bioreactor. This means of control requires daily measurement of metabolite concentrations. Adjustments of perfusion are often made only after cell damaging changes have occurred in the culture medium. Control of pH by addition of a concentrated buffer solution can also cause cell damage as locally high buffer concentrations and pH are produced at the point where the buffer enters the culture medium.

A more rational means of controlling perfusion rates would tie an indicator of cellular metabolic rate to the rate at which fresh nutrients are added and waste products are removed. Animal cells in culture metabolize glutamine to ammonia and glutamate, glucose to lactic acid and pyruvate. The pyruvate is then metabolized to CO₂ and H₂O. Both the lactic acid and the CO₂ reduce the pH of the bicarbonate buffered culture fluid. Typically, commercially available media contain standard amounts of nutrients and bicarbonate buffer. In order to be used in the scheme described here, these media may have to be altered by the addition of glucose, glutamine or bicarbonate to provide appropriate amounts of nutrients and buffering capacity and sufficient perfusion volume to remove waste products. Each cell type and set of culture conditions have their own requirements and exact concentrations of the additives have to be determined experimentally.

Therefore, the primary object of this invention is to provide a method for automating the control of perfusion rates in bioreactor cultures of animal cells grown either in suspension or attached to microcarriers.

Another object of the invention is the elimination of separate buffer addition to the bioreactor for the control of pH in animal cell cultures.

### BRIEF DESCRIPTION OF THE DRAWINGS

FIG. 1 is a diagrammatic illustration of the invention as it applies to bioreactor cultures of cells grown on microcarriers.

FIG. 2 is a diagrammatic illustration of the invention as it applies to bioreactor cultures of cells grown in suspension.

### DETAILED DESCRIPTION OF THE INVENTION

The invention is a method for controlling perfusion rates in a continuous bioreactor culture of animal cells. The animal cells may be used for the production of viral vaccine, monoclonal antibodies or recombinant proteins. A pH probe penetrates into the bioreactor and measures the pH of the culture medium in the bioreactor. The signal from the pH probe is sent, by way of a controller, to a pump which controls the addition of a fresh culture medium to the bioreactor and/or removal of spent culture medium containing waste products. The fresh medium contains a mixture of standard cell culture medium components plus experimentally determined amounts of glucose, glutamine and bicarbonate, such that perfusion, as initiated by the signal from the pH probe when the pH falls below the setpoint and stops when pH rises above the setpoint, maintains glucose and glutamine at levels which promote cell growth and viability and removes waste products, specifically lactic acid, CO₂ and ammonia, such that they do not attain concentrations which reduce cell growth and viability.

Bioreactor cultures which employ perfusion of fresh medium into the bioreactor and remove an equivalent amount of spent culture medium offer the advantage of continuously providing nutrients to the cells and removing potentially toxic waste products. The results are increased cell yields per culture and increased time of productive culture. In addition, the costs of setup and cleaning are reduced since fewer cultures need be run to produce the same amount of product.

Monitoring pH in the bioreactor by means of a pH probe reflects the metabolic rate of the cells in the bioreactor. The signal from the pH probe can activate a pump which feeds fresh medium containing nutrients and a bicarbonate buffer, when the pH drops below a predetermined set point. In this way perfusion becomes automated and is responsive to the metabolic activity of the cells in the bioreactor. This scheme eliminates the need for 1) a separate buffer addition and 2) manual adjustment of the perfusion rate. It also reduces the number of pumps needed and the number of penetrations into the bioreactor, making setup simpler and less prone to contamination.

The invention is predicated upon investigation of the required perfusion rates and bicarbonate buffer additions for maintaining healthy cultures of animal cells in stirred tank bioreactors. By altering the amount of bicarbonate in the perfusion medium, we have discovered that the requirement of a separate addition, to the bioreactor, of concentrated bicarbonate buffer can be eliminated and that by balancing the concentrations of glucose and glutamine with bicarbonate in the perfusion medium, the perfusion rate could be determined by the pH of the culture.

As a consequence of the foregoing observations and studies, the method of the present invention takes into account the needs of the cells for stable control of pH, constant levels of energy sources and nutrients, and low levels of waste products. The pH is precisely controlled by increased perfusion and resultant addition of bicarbonate from the perfusion medium, if the pH falls below setpoint, and by addition of CO₂, if the pH rises above setpoint. The levels of glucose and glutamine in the culture fluid are thus determined by their concentrations in the perfusion medium and the rate of perfusion as controlled by the need to increase the pH of the culture fluid as a further result of the metabolic rate of the cells. The amounts of glucose, glutamine and bicarbonate in the perfusion medium are determined experimentally and individually for each cell type and culture protocol.

By virtue of controlling perfusion according to the invention, cultures of anchorage dependent cells on microcarriers, can be grown for a standard 7 day period without manual alteration of perfusion or the addition of concentrated bicarbonate. Cell growth of over 4 population doublings to over 3.5 x 10⁶ cells/ml is achieved. The medium is then infected with virus for the production of vaccine.

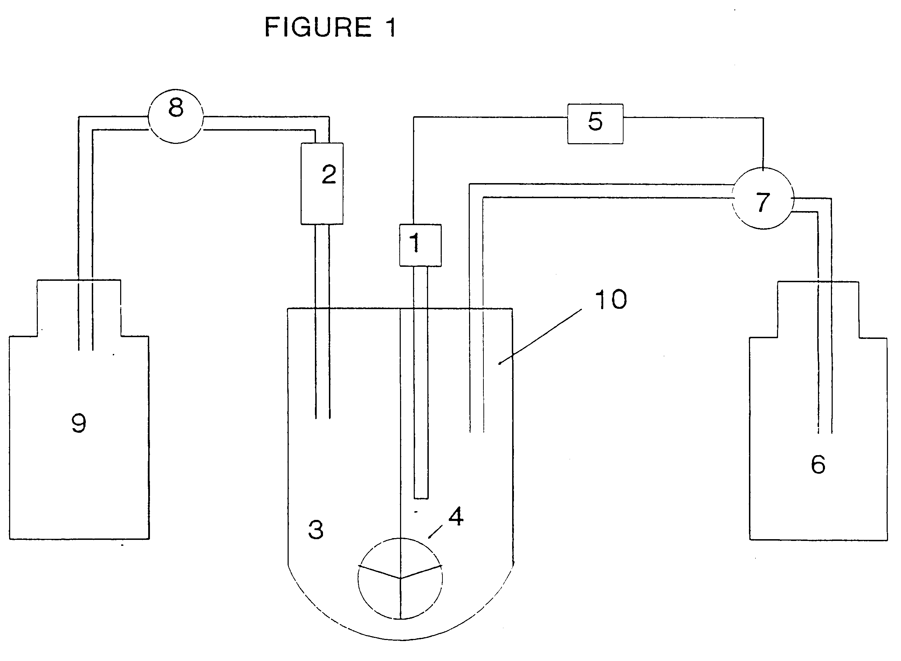

In the first preferred embodiment of the invention and with reference to FIG. 1 and cultures of cells grown on microcarriers, there is shown a stirred tank culture vessel (3), generally constructed of glass or stainless steel. Within the culture vessel is a draught tube settling chamber assembly (2), a motor driven impeller (4), a pH probe (1), and an inlet tube (10). The pH probe is connected to a pH controller (5) which is in turn connected to a pump (7). The pump feeds fresh medium containing experimentally determined concentrations of glutamine, glucose and bicarbonate, from the medium reservoir (6) into the bioreactor (3) through the inlet tube (10). Culture fluid is removed by a pump (8) from the bioreactor through the draught tube settling chamber assembly (2) and into the harvest reservoir (9). During perfusion, the perfusion out pump (8) pumps continuously to remove culture fluid as it rises above the preset level determined by the level of the draught tube settling chamber assembly (2) in the bioreactor (3). The pH probe (1) is submerged in the culture fluid and when the pH of the culture fluid drops below a preset value it sends a signal to a controller (5) which activates a pump (7) which pumps fresh medium from the medium reservoir (6) through the inlet tube (10) into the bioreactor (3). The motor driven impeller (4) maintains the cell laden microcarriers in suspension and mixes the fresh medium into the culture fluid. As an example of the first embodiment, Vero cells attached to Cytodex I microcarriers are grown in the bioreactor in Minimal Essential Medium (MEM) containing 10% fetal bovine serum, 2.5 g/l glucose, 2 mM glutamine and 1.68 g/l sodium bicarbonate. Cell density at the start of the culture is 2 x 10⁸ cells/liter and the microcarrier density is 7.5 g/liter. The perfusion medium is MEM containing 2.5 g/l glucose, 2 mM glutamine and 1.68 g/l sodium bicarbonate. During the standard 7 day culture period the cells grow to a density of 3.7 x 10⁹ cells/ml, the glucose concentration remains at or above 1 g/l, the glutamine concentration remains at or above 1 mM and the pH is maintained at 7.3. The ammonia concentration remains below 2 mM and the lactate concentration remains below 20 mM. The cells from the culture are infected with Herpes Simplex virus for the production of vaccine.

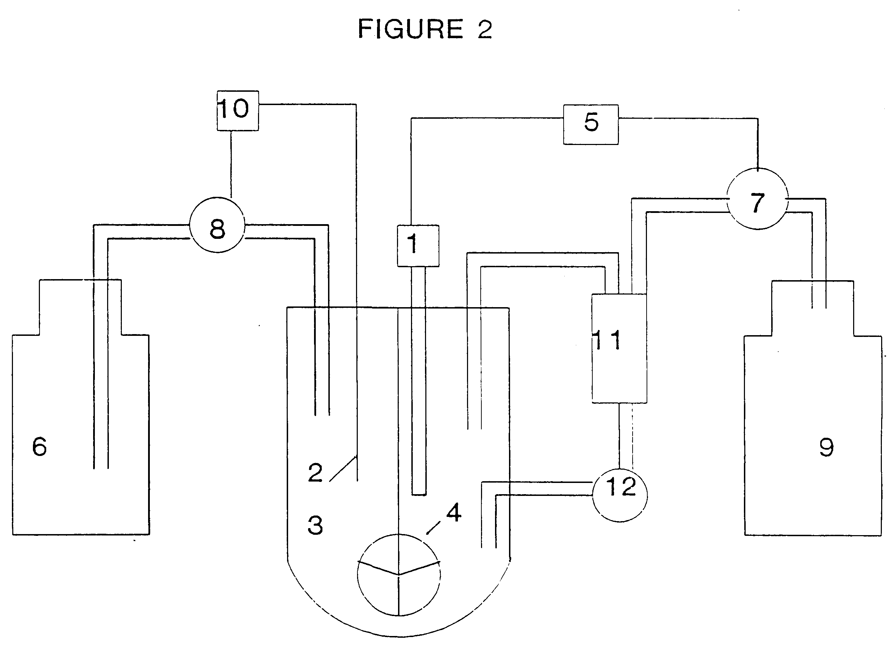

In the second preferred embodiment of the invention and with reference to FIG. 2 and cultures of cells grown in suspension, cells are continuously recirculated through a tangential flow type submicron filter (11) by a pump (12). The pH probe (1), submerged in the culture fluid in the vessel (3) sends its signal by way of the controller (5) to a pump (7) which removes culture fluid from the vessel (3) through the submicron filter such that cells remain in the fluid which returns to the vessel (3) and the removed culture fluid travels to a harvest bottle (9). Fresh medium is added from a medium bottle (6) as controlled by a signal from a level probe (2) and level controller (10) to a pump (8). During perfusion the recirculation pump (12) runs continuously pumping cells and culture fluid into the filter. When the pH drops below setpoint the perfusion out pump (7) is activated, drawing a portion of the culture fluid through the filter, while the cells and remaining culture fluid return to the vessel (3). Fresh medium from the medium bottle (5) is added when the pump (8) is activated by a signal from the level controller as the level in the culture vessel (3) drops below the level probe (2).

As an example of the second embodiment of the invention, hybridoma cells are grown in suspension in a protein free hybridoma medium containing 10% fetal bovine serum. Cell density at the start of the culture is 2 X 10⁸ cells/l. During the growth phase of the culture the perfusion medium is protein free hybridoma medium containing 5% fetal bovine serum 6 mM glutamine, 6 g/l glucose, and 3 g/l bicarbonate. The pH setpoint is 7.2 during this phase. After 6 days when the cells reach 5 x 10⁹ cells/l, the pH setpoint is reduced to 7.0 and the fetal bovine serum is eliminated from the perfusion medium. Cell density is maintained between 5 and 10 x 10⁹ cells/l for 14 days. The entire culture period is 20 days. Glucose remains above 1 g/l, glutamine, above 1 mM, ammonia below 3.5 mM and lactate below 30 mM. Antibody produced by this culture is used in the affinity purification of a glycoprotein.

Although the invention has been described with reference to particular features and exemplary parameters, these are intended to illustrate rather than limit the scope of the invention as defined in the appended claims.

## Claims

1. A method for controlling perfusion rates in a continuous bioreactor culture of animal cells grown in medium requiring pH maintenance which comprises:
(a) adding fresh media enriched with glutamine, glucose and bicarbonate to the reaction medium in the bioreactor when the reaction medium pH drops below setpoint;
(b) discontinuing the addition of the enriched fresh medium when the reaction medium pH reaches setpoint; and
(c) repeating steps (a) and (b) on a continuous basis to maintain the desired setpoint pH.

2. A method according to Claim 1 wherein the cells are mammalian cells infected with virus for the production of vaccines.

3. A method according to Claim 2 wherein the culture medium is maintained between a pH of 6.8 and 7.6.

4. A method according to Claim 3 wherein the mammalian cells are Vero cells for the production of inactivated poliovirus vaccine and oral polio vaccine which comprises:
(a) adding the fresh medium enriched with glucose to about 2.5 grams per liter, and bicarbonate of 1.68 g/l to obtain a setpoint of 7.3;
(b) discontinuing the addition of the enriched fresh medium until the pH of the reaction medium falls below 7.3; and
(c) repeating steps (a) and (b) on a continuous basis to maintain a setpoint pH of 7.3.

5. A method according to Claim 1 wherein the cells are hybridoma cells.

6. A method according to claim 5 wherein the culture media is maintained between a pH of 6.8 and 7.6.

7. A method according to Claim 6 wherein the cells are hybridoma cells for the production of Herpes vaccine which comprises:
(a) adding the fresh medium enriched with about 6 grams of glucose per liter, glutamine to about 5 mM and bicarbonate of 3 g/l to obtain a setpoint pH between 7.0 and 7.4;
(b) discontinuing the addition of the enriched fresh medium until the pH of the reaction medium falls below setpoint and
(c) repeating steps (a) and (b) on a continuous basis to maintain a setpoint pH between 7.0 and 7.4.

8. A method according to Claim 1 wherein the cells are mammalian cells that are transformed for the production of recombinant protein.

9. A method according to Claim 4 wherein the mammalian cells are of avian, simian or human origin.

10. A method according to Claim 7 wherein the hybridoma cells are of simian, murine or human origin.
